# EUROPEAN PATENT SPECIFICATION

(11) **EP 2 966 171 B1**
(45) Date of publication and mention of the grant of the patent: **09.10.2019**
(21) Application number: 13722787.2
(22) Date of filing: 06.02.2013
(51) Int. Cl.: C12P 5/02, C12P 7/06, C12P 7/64, C12P 13/04

(54) **METHOD FOR THE VALORISATION OF PHOTOSYNTHETIC MICROORGANISMS FOR INTEGRAL USE OF BIOMASS**
VERFAHREN ZUR VERWERTUNG PHOTOSYNTHETISCHER MIKROORGANISMEN ZUR INTEGRIERTEN VERWENDUNG VON BIOMASSE
PROCESSUS DE VALORISATION DES MICROORGANISMES PHOTOSYNTHÉTIQUES POUR L'UTILISATION INTÉGRALE DE LA BIOMASSE

(43) Date of publication of application: 13.01.2016
(73) Proprietor: Algaenergy S. A., 28108 Alcobendas (Madrid) (ES); Universidad de Almeria, 04120 Cañada de San Urbano, Almeria (ES)
(72) Inventor: ORTIZ MONTOYA, Erika Yuliana, E-28108 Alcobendas (Madrid) (ES); LLAMAS MOYA, Bernardo, E-28108 Alcobendas (Madrid) (ES); MOLINA GRIMA, Emilio, E-04120 Almería (ES); GARCÍA CUADRA, Francisco, E-04120 Almería (ES); FERNANDEZ SEVILLA, José, E-04120 Almería (ES); ACIEN FERNANDEZ, Francisco Gabriel, E-04120 Almería (ES)
(74) Representative: Monzón de la Flor, Luis Miguel
(86) International application number: PCT/ES2013/070064
(87) International publication number: WO 2014/122331

(56) References cited:
- DE-A1-102010 001 072
- US-A1- 2007 048 859
- US-A1- 2008 160 593
- US-A1- 2012 270 304
- Danquah, M. et al.,: "Analysis of process configurations for bioethanol production from microalgal biomass"; "Chapter 20" In: Dr. Shahid Shaukat (Ed.): "Progress in Biomass and Bioenergy Production", 2011, XP002713870, ISBN: 978-953-307-491-7 pages 395-408, DOI: 10.5772/17468, page 400, paragraph 1 - page 406, paragraph 1; figure 2; table 4
- MORRIS H J ET AL: "Utilisation of Chlorellavulgaris cell biomass for the production of enzymatic protein hydrolysates", BIORESOURCE TECHNOLOGY, vol. 99, no. 16, 1 November 2008 (2008-11-01), pages 7723-7729, XP022735260, ELSEVIER BV, GB ISSN: 0960-8524, DOI: 10.1016/J.BIORTECH.2008.01.080 [retrieved on 2008-03-24]
- Schneider, R.S.C. et al.: "Potential Production of Biofuel from Microalgae Biomass Produced in Wastewater"; "Chapter 1" In: Prof. Zhen Fang (Ed.): "Biodiesel-Feedstocks, Production and Applications", 2012, XP002713871, ISBN: 978-953-51-0910-5 pages 3-24, DOI: 10.5772/52439, page 3, last paragraph - page 4, paragraph 3; figures 1,3 page 8, paragraph 2 - page 16, paragraph 3
- HANNON, M. ET AL.,: "Biofuels from algae: challenges and potential", NIH Public Access Author Manuscript , September 2010 (2010-09), pages 1-35, XP055081834, Retrieved from the Internet: URL:http://www.ncbi.nlm.nih.gov/pmc/articl es/PMC3152439/ [retrieved on 2013-09-27]
- WILLIAMS P J LE B ET AL: "Microalgae as biodiesel and biomass feedstocks: review and analysis of the biochemistry, energetics and economics", ENERGY & ENVIRONMENTAL SCIENCE, vol. 3, 1 February 2010 (2010-02-01), pages 554-590, XP009149363, ROYAL SOCIETY OF CHEMISTRY, UK ISSN: 1754-5692, DOI: 10.1039/B924978H [retrieved on 2010-03-22]
- HARUN R ET AL: "Bioprocess engineering of microalgae to produce a variety of consumer products", RENEWABLE AND SUSTAINABLE ENERGY REVIEWS, vol. 14, no. 3, 1 April 2010 (2010-04-01), pages 1037-1047, XP026867884, ELSEVIERS SCIENCE, NEW YORK, NY, US ISSN: 1364-0321 [retrieved on 2009-11-24]
- MARTÍN, M AND GROSSMANN, I.E: "Optimal engineered algae composition for the integrated simultaneous production of bioethanol and biodiesel", AICHE JOURNAL , vol. 59, no. 8 2 April 2013 (2013-04-02), pages 2872-2883, XP002713872, DOI: 10.1002/aic.14071 Retrieved from the Internet: URL:http://onlinelibrary.wiley.com/doi/10. 1002/aic.14071/pdf [retrieved on 2013-09-24]
- RASHID, N. ET AL.M: "Recycling and reuse of spent microalgal biomass for sustainable biofuels", BIOCHEMICAL ENGINEERING JOURNAL , vol. 75 June 2013 (2013-06), pages 101-107, XP055081838, Retrieved from the Internet: URL:http://dx.doi.org/10.1016/j.bej.2013.0 4.001 [retrieved on 2013-09-27]

## Description

### OBJECT OF THE INVENTION

This invention is included within the biofuel production from photosynthetic microorganisms. More specifically, it pertains to the field of obtaining compounds of commercial interest from large-scale outdoor biomass production for the biofuels market.

The object of the patent is an optimised process for obtaining products of interest from photosynthetic microorganisms, including the sequence of stages and the optimum operating conditions to maximise the amount and quality of the products obtained, achieving integral use of biomass in a sustainable, economically viable manner.

The photosynthetic microorganisms valorization process for integral use of biomass of the invention, is characterised in that it includes the following stages:
- An initial stage whereby microalgal biomass (1) is placed in an agitated tank in which cellulolytic enzymes (2) are used to produce liquefaction of the sugars (3), rupturing the cell walls and releasing the soluble carbohydrates, with the rest of the components remaining in the residual biomass; the mixture obtained is separated using a filtering and/or centrifuging process (4), giving rise to a clarified solution (5) rich in carbohydrates and a concentrate containing the residual biomass (9);
- A stage consisting of production of bioethanol and other derived products such as lactic acid from the aforementioned solution (5) by means of saccharification (6) of the carbohydrates, followed by alcoholic fermentation (7) with yeast to transform the monosaccharides into bioethanol (8) with a high yield fermertation process;
- A stage consisting of production of peptide and amino acid concentrates (10) using proteolytic enzymes (12) which break down and solubilize the proteins in an enzymatic hydrolysis process (11), transforming the proteins into peptides and amino acids with a lower molecular weight that can be used for foodstuffs or as high-value agricultural fertilizers. The reaction mixture obtained is then separated using a filtering and/or centrifuging process (13), giving rise to a solution (14) rich in peptides and amino acids and a concentrate containing the residual biomass (15);
- A stage consisting of biodiesel production from the aforementioned concentrate (15), involving transesterification (17) of the fatty acids in the biomass with sulphuric acid as a catalyser and methanol as a reagent (16), to obtain the respective methyl esters making it up. When the reaction is complete, the biodiesel (20) is extracted from the reaction mixture using hexane (18), which is then evaporated to obtain the biodiesel (20) and
- A stage consisting of biogas production, whereby the residual biomass (21) resulting from the previous stage is subjected to an anaerobic digestion process (22) by the action of methanogenic bacteria, in order to produce biogas (23) from the resulting methanol-free and hexane-free reaction mixture (21), whereby the residual carbonaceous matter contained in the same is transformed into methane, hydrogen and CO2.

### BACKGROUND OF THE INVENTION

Microalgae are extremely useful organisms, having a huge number of beneficial applications in a variety of fields such as pharmaceutical and nutraceutical compound production, human and animal feeds, waste water treatment and biofuel production (Spolaore, et al. 2006). Using microalgae as a source of biofuel has numerous advantages, including the fact that microalgal cultures can achieve much higher production levels than traditional crops, resulting in higher CO₂ fixing and a larger amount of biomass produced. Microalgal cultures also have less water requirements and do not compete with the traditional crops, as they do not require fertile soil or fresh water. Finally, they are a legally accepted source of biofuels and, given their CO₂ fixing properties, they are nowadays one of the most sustainable sources for obtaining third-generation biofuels (Chisti 2007).

In order to produce biofuels from microalgae, large-scale production systems are required, covering huge extensions of hundreds of hectares to make the necessary investment profitable, in addition to industrial facilities of a sufficient size. The production stage involves use of low-cost materials, highly efficient technologies and the use of residues from other industries such as CO₂ and effluents contaminated with nitrogen and phosphorus, to achieve a sustainable and economically viable production process (Olguin 2012; Pittman, et al. 2011). However, regardless of the production process installed, a biomass utilisation process is also essential, enabling the biomass to be used in its totality, obtaining the maximum amount of products of proven quality. Only integral use of the biomass and its transformation into valuable products can make the overall production + valorization process economically viable (Acién, et al. 2012; Molina Grima, et al. 2003; Singh, et al. 2011).

It has been described that microalgae can be used to produce bioethanol, due to their high carbohydrate content (Harun and Danquah 2011). To achieve this, the carbohydrates contained in the biomass need to undergo a process of liquefaction and subsequent saccharification to obtain fermentable monosaccharides. The conditions proposed for performing this liquefaction and saccharification process are generally extreme, working at a pH of between 1 and 3 and temperatures between 100 and 250°C (Harun and Danquah 2011). The conditions for fermentation with yeasts are milder, and should be adapted to the optimum of the yeast employed, with temperatures varying between 20 and 50°C and the pH between 7 and 9 (Khaw, et al. 2006; Mesa, et al. 2010). The greatest disadvantage of these processes is that they do not make use of the rest of the compounds present in the biomass, as during the liquefaction and saccharification process both the lipids and the proteins contained in it are destroyed, producing intermediates that may be toxic for the biogas production processes under anaerobic conditions.

It has also been described that microalgae can be used to produce amino acid concentrates (Romero Garcia, et al. 2012). Microalgae have a high protein content, up to 50% dry weight, with amino acid profiles of great interest for their nutritional quality (Rebolloso Fuentes, et al. 2000; Rebolloso-Fuentes, et al. 2001). Amino acid hydrolysates can be used in human and animal feeds (Morris 2008), or as biofertilizers (Ördög, et al. 2004). They can be obtained chemically or enzymatically, the latter system being recommendable in order to maintain the optimal properties of the amino acids contained in the biomass. Prior cell rupture and amino acid release implies loss of carbohydrates, making it impossible to subsequently produce bioethanol from the carbohydrates contained in the biomass using processes of this type.

As regards biodiesel production, microalgae can be used to produce oils and subsequently transesterify them, as is habitually done with vegetable oils. Alternatively, direct transesterification of the complete biomass can be performed directly (Belarbi, et al. 2000) The oil extraction and subsequent transesterification is hindered by the presence of water in the microalgal biomass, and highly polar solvents and harsh extraction conditions must therefore be used. This also results in complex lipids, due to the polar nature of most of the lipids contained in the microalgal biomass. Moreover, the direct transesterification is performed at a high temperature and under high acidity conditions (temperatures of 100-300°C and a pH of 2-4), in the presence of methanol and with sulphuric acid as a catalyser, causing the total denaturation of all the compounds in the biomass. As with liquefaction and saccharification of sugars, this means that the rest of the compounds in the biomass are not used, and this can jeopardise biogas production due to the formation of toxic compounds.

Whatever the photosynthetic microorganism utilisation process used, the following considerations must be taken into account to achieve maximum performance:
- Photosynthetic microalgal biomass is a heterogeneous material, consisting of biomass and water, as available harvesting processes achieve maximum dry material concentrations of 20-30%, the rest being water.
- The water can be eliminated through drying operations, but this has a negative effect on both energy consumption and economic viability, as drying is a costly process. The valorization processes should therefore be performed on wet biomass.
- Photosynthetic microorganisms cells have a tough cell wall that must be broken to release the intracellular content and to obtain the products of interest. Use of harsh chemical products or extreme pressure and temperature conditions are penalised as they reduce the sustainability and economic viability of any process.
- All processes of extraction or transformation of the compounds found in biomass affect the rest of the compounds, and thus the performance or quality of the subsequent products is always affected by the previous biomass treatments.
- All processes used to obtain compounds from biomass create residue, whose composition and conditions will be determined by the sequence of previous stages. The dual objective of eliminating this residue and making use of it is essential for improving the overall viability of the process developed.

### References

Acién FG, Fernandez JM, Magan JJ, Molina E. 2012. Production cost of a real microalgae production plant and strategies to reduce it. Biotechnol Adv 30:1344-1353
Belarbi E, Molina E, Chisti Y. 2000. A process for high yield and scaleable recovery of high purity eicosapentaenoic acid esters from microalgae and fish oil. Process Biochem 35:951-969.
Chisti Y. 2007. Biodiesel from microalgae. Biotechnol Adv 25:294-306.
Harun R, Danquah MK. 2011. Influence of acid pre-treatment on microalgal biomass for bioethanol production. Process Biochem 46:304-309.
Khaw TS, Katakura Y, Koh J, Kondo A, Ueda M, Shioya S. 2006. Evaluation of performance of different surface-engineered yeast strains for direct ethanol production from raw starch. Appl Microbiol Biotechnol 70:573-579.
Mesa L, Gonzalez E, Cara C, Ruiz E, Castro E, Mussatto SI. 2010. An approach to optimization of enzymatic hydrolysis from sugarcane bagasse based on organosolv pretreatment. J Chem Technol Biotechnol 85:1092-1098.
Molina Grima E, Belarbi E-, Acién Fernandez FG, Robles Medina A, Chisti Y. 2003. Recovery of microalgal biomass and metabolites: Process options and economics. Biotechnol Adv 20:491-515.
Morris HJ. 2008. Cuba: Protein hydrolysates derived from green microalgae. Ind. Bioprod 30:7-14.
Olguin EJ. 2012. Dual purpose microalgae-bacteria-based systems that treat wastewater and produce biodiesel and chemical products within a Biorefinery. Biotechnol Adv 30:1031-1046.
Ördög V, Stirk WA, Lenobel R, Bancirova M, Strnad M, Van Staden J, Szigeti J, Németh L. 2004. Screening microalgae for some potentially useful agricultural and pharmaceutical secondary metabolites. J Appl Phycol 16:309-314.
Pittman JK, Dean AP, Osundeko O. 2011. The potential of sustainable algal biofuel production using wastewater resources. Bioresour Technol 102:17-25.
Rebolloso Fuentes MM, Acién Fernandez GG, Sanchez Perez JA, Guil Guerrero JL. 2000. Biomass nutrient profiles of the microalga Porphyridium cruentum. Food Chem 70:345-353.
Rebolloso-Fuentes MM, Navarro-Pérez A, Garcia-Camacho F, Ramos-Miras JJ, Guil-Guerrero JL. 2001. Biomass nutrient profiles of the microalga Nannochloropsis. J. Agri Food Chem 49:2966-2972.
Romero Garcia JM, Acién Fernandez FG, Fernandez Sevilla JM. 2012. Development of a process for the production of I-amino-acids concentrates from microalgae by enzymatic hydrolysis. Bioresour Technol 112:164-170.
Singh A, Nigam PS, Murphy JD. 2011. Mechanism and challenges in commercialisation of algal biofuels. Bioresour Technol 102:26-34.
Spolaore P, Joannis-Cassan C, Duran E, Isambert A. 2006. Commercial applications of microalgae. J. Biosc Bioeng 101:87-96.

### DESCRIPTION OF THE INVENTION

This invention refers to an optimised process for obtaining products of interest from photosynthetic microorganisms, including the sequence of stages and the optimum operating conditions to maximise the amount and quality of the products to be obtained, achieving integral biomass use, the entire process being technically feasible and scalable, but above all sustainable and economically viable.

The proposed process consists of the following stages:
- An initial stage consisting of carbohydrate extraction using cellulolytic enzymes which rupture the cell wall and release the soluble carbohydrates, with the rest of the components remaining in the residual biomass. This enables liquefaction of most of the carbohydrates in the biomass, which then go on to serve as a raw material for the subsequent processes, e.g. lactic acid or bioethanol. For this purpose, the mixture obtained must be separated by a filtering and/or centrifugation process, giving rise to a solution rich in carbohydrates and a concentrate containing the residual biomass.
- A stage consisting of bioethanol production from the said solution, by means of saccharification of the carbohydrates under moderate acid conditions (pH = 1.5-2.5) and temperature (95-115°C), followed by fermentation with yeast of the *Saccharomyces* genus for 24 h, in order to transform the monosaccharides into bioethanol with a high yield fermentation process. The yeast can be recovered for reuse by filtering and/or centrifugation, with bioethanol and unmetabolised sugar residues remaining as content in the liquid phase. The bioethanol produced can be separated by vacuum distillation and subsequent desiccation using molecular sieves.
- A stage consisting of production of peptide and amino acid concentrates using proteolytic enzymes that rupture the proteins, solubilizing them and transforming them into peptides and amino acids of a lower molecular weight that can be used in foodstuffs or as high-value agricultural fertilizers. The process is performed using the residual biomass from the previous stage in a stirred-tank reactor at a controlled pH and temperature (pH = 7.7-8.1, T= 40-55°C) for 4 h, under gentle conditions, without using toxic products or harsh conditions. The mixture obtained is then separated using a filtering and/or centrifuging process, giving rise to a solution rich in peptides and amino acids and a concentrate containing the residual biomass. The amino acid-peptide solution is then stabilised by acid or alkaline pH adjustment to prevent decomposition.
- A stage consisting of biodiesel production from the above concentrate, whereby transesterification of the fatty acids in the biomass is performed, using sulphuric acid as a catalyser and methanol as a reagent, to obtain the respective methyl esters making it up. The reaction is performed under gentle conditions without prior biomass drying, using sulphuric acid at 2% and at a temperature of 90-110°C, for 2 h. On completion of the reaction, the biodiesel is extracted from the mixture using hexane, which is then evaporated to obtain the biodiesel. The unused methanol is similarly removed from the reaction mixture by vacuum distillation. In this way both the solvents are recovered and their consumption is minimised, thus improving both the sustainability and the economic viability of the process.
- A stage consisting of biogas production from the resulting methanol-free and hexane-free reaction mixture, transforming its residual content of carbonaceous matter into methane, hydrogen and CO₂ by the action of methanogenic bacteria. The process takes place in sealed tanks under anaerobic conditions at temperatures of 30-40°C, with a residence time of 5-8 days.

### PREFERRED EMBODIMENT OF THE INVENTION

The proposed process may be performed partially or in its entirety, but the order and operating conditions indicated as optimum to maximise use of the biomass and all its components must always be observed. Whether a particular stage is performed or not will be determined by the amount of biomass compound to be utilised (carbohydrates, proteins, lipids) and the interest or value of the product to be obtained (bioethanol, peptides and amino acids, biodiesel, biogas, lactic acid, high-value fatty acids). It should however be stressed that failure to perform one of the stages would hinder rather than enhance performance of the subsequent stages, as removal of the different biomass compounds firstly reduces the presence of compounds that could interfere with the subsequent reactions, and secondly increases the richness of the products remaining in the biomass, thus improving the performance of the subsequent stages.

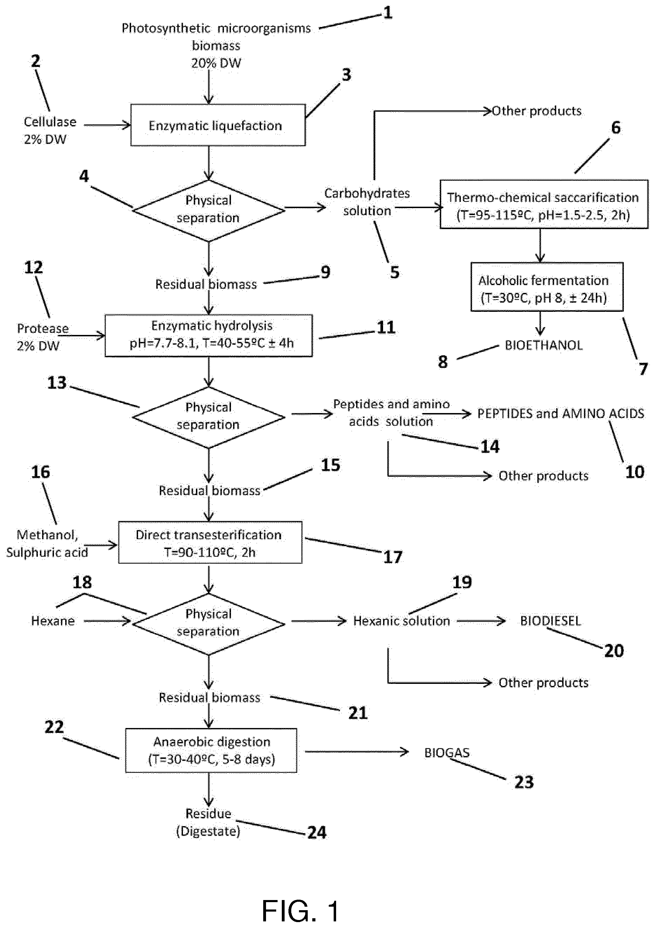

The preferred embodiment of the invention is shown in Figure 1. It can be observed that the photosynthetic microorganism biomass (1) obtained from the culture system and harvesting is conditioned to achieve a dry matter concentration of 15-20%. The photosynthetic microorganism mixture attained is placed in a stirred-tank reactor, adjusting the temperature to the optimum operating conditions of the cellulolytic enzymes (using cellulase), adding an amount of enzyme (2%) (2) and leaving it to act for 2 h. During this time, liquefaction of the sugars occurs (3) and the carbohydrates in the biomass are released. Depending on the carbohydrate content of the biomass, the concentration of carbohydrates in the mixture can reach up to 60 g/l, although it rarely exceeds 30 g/l. When the reaction time is complete, the mixture is removed from the stirred-tank reactor and filtered/centrifuged (4) to separate the solution containing the carbohydrates from the remaining biomass. The carbohydrates solution (5) is then subjected to a saccharification process by acid-thermal treatment at pH= 1.5-2.5 and T= 95-115°C for 2 h (6). This is performed either in the same tank or in an additional one, with agitation. After this time, the aforementioned concentrate is cooled to 30°C and the pH (7) adjusted to the optimum operating conditions of the *Saccharomyces* genus yeast. When the conditions have stabilised, 2% of yeast is added, leaving it to ferment with agitation and no aeration, under anoxic conditions, for approximately 24 h. After this time, the free sugars will have been transformed into CO₂ and bioethanol (8), with the yeast remaining in suspension. The yeast can be recovered by filtering/centrifuging and recirculated to the process for a new batch. The solution contains the bioethanol to be recovered by vacuum distillation (P=10-20 mbar, T=30-40°C), to obtain an azeotrope containing 4% water and 96% bioethanol. The water may be removed if necessary, using molecular sieves, for use as a direct biofuel mixed with gasoline.

In the preferred embodiment, the residual biomass (9) resulting from the sugar extraction process can be used to obtain peptide and amino acid concentrates (10). If the previous stage is not performed, the biomass may also be used directly to obtain peptide and amino acid concentrates, but in this case the solution will also contain carbohydrates which will not be exploited. To obtain peptide and amino acid concentrates, the wet biomass with 15-20% dry matter is placed in an stirred-tank reactor with pH and temperature control. The pH and temperature are adjusted to the optimum conditions of the enzyme (12) to be used, preferably endo- and exo-proteases, with pH= 7.7-8.1 and T= 40-55°C, in an enzymatic hydrolysis process (11). The first enzyme is added, followed by the second enzyme 2 h later, both in the same amount, 2%. The process is completed in approximately 4 h, after which most of the protein fraction will have been hydrolysed and the resulting peptide and amino acids dissolved in the liquid phase. To separate them, the reaction mixture is centrifuged (13), resulting in a solution (14) that contains the said peptides and amino acids (10) and a residual biomass (15). The peptides and amino acids solution (14) must be stabilised to prevent rapid decomposition, as it is a perfect breeding ground for bacteria and yeast. The simplest solution is therefore to adjust the pH of the clarified solution to values preventing the growth of bacteria and yeasts. Good results are obtained with both acid and alkaline pH. Stabilisation at extreme pH values is not recommended, as in addition to being costlier and more hazardous - as the materials are more corrosive - the peptides and amino acids themselves may be denatured, losing part of their properties.

In the preferred embodiment, when the process for obtaining bioethanol and peptides and amino acids concentrate is complete, the resulting residual biomass (15) may be used to produce biodiesel. If the previous stages have not been performed, the biomass may also be subjected to the biodiesel production process, although the process will not perform as well and the quality of the resulting products will be lower. It is therefore recommended to carry out the previous stages, increasing the amount of products obtained from the same biomass. To perform this process, the wet biomass, with a dry matter content of 15-20%, is placed in an stirred-tank reactor, adding sulphuric acid to 2% as a catalyser and methanol (16) as a reagent to an amount of 1 L for each litre of wet biomass. It is then subjected to a transesterification process (17). The mixture is heated to a temperature of 90-110°C and left to react for 2 h. As organic solvents are being worked with in this case, the reactor used must be sealed and must comply with applicable ATEX standards. After this time, hexane is added to the resulting mixture and agitated (18) for 0.5 h, so that the methylic esters formed are transferred from the aqueous-methanolic phase to the hexanic phase. The hexanic phase is removed and the hexane is recovered via vacuum distillation (19) (P=10-20 mbar, T=30-40°C), obtaining liquid biodiesel (20). The aqueous-methanolic phase is treated to recover the excess methanol added, for which purpose is also submitted to vacuum distillation (P=10-20 mbar, T=30-40°C) until the methanol is completely recovered. Both the methanol and the hexane are thus recovered and recirculated to the process. The residual biomass (21) therefore remains, in liquid phase, together with the reaction residues from all the previous stages. This biomass can be used to produce biogas.

In the preferred embodiment, on completion of the stages consisting of production of bioethanol (8), peptide and amino acids (10) and biodiesel (20), the resulting residual biomass (21) is subjected to a process of anaerobic digestion (22) to produce biogas (23). If the previous stages are not performed, the biomass may be subjected to anaerobic digestion to produce biogas, although the process does not perform as well as the biomass is not sufficiently broken down, the nitrogen content is excessive and the chemical structure of the compounds present is too complex for the methanogenic bacteria to efficiently degrade the organic material. Biogas production is achieved by placing the wet biomass, with a dry matter content of 15-20%, in a continuous anaerobic digester, with a residence time of 5-8 days. The anaerobic digester is a sealed reactor that must previously be stabilised at a suitable temperature and pH, with no air inlet, and with bacterial flora adapted to the substrate to be metabolised. The biogas formed is removed through the top of the tank and the digestate (24) is removed through the bottom of the tank. With a minimal volume, it can also be used as a fertilizer for its high ammonium and phosphorus content and low COD.

## Claims

1. Photosynthetic microorganisms valorization process for integral use of biomass, **characterised in that** it includes the following stages:
- An initial stage whereby microalgal biomass (1) is placed in an agitated tank in which cellulolytic enzymes (2) are used to produce liquefaction of the sugars (3), rupturing the cell walls and releasing the soluble carbohydrates, with the rest of the components remaining in the residual biomass; the mixture obtained is separated using a filtering and/or centrifuging process (4), giving rise to a clarified solution (5) rich in carbohydrates and a concentrate containing the residual biomass (9).
- A stage consisting of production of bioethanol and other derived products such as lactic acid from the aforementioned solution (5) by means of saccharification (6) of the carbohydrates, followed by alcoholic fermentation (7) with yeast to transform the monosaccharides into bioethanol (8) with a high yield fermentation process.
- A stage consisting of production from the residual biomass (9) of peptide and amino acid concentrates (10) using proteolytic enzymes (12) which break down and solubilize the proteins in an enzymatic hydrolysis process (11), transforming the proteins into peptides and amino acids with a lower molecular weight that can be used for foodstuffs or as high-value agricultural fertilizers. The reaction mixture obtained is then separated using a filtering and/or centrifuging process (13), giving rise to a solution (14) rich in peptides and amino acids and a concentrate containing the residual biomass (15).
- A stage consisting of biodiesel production from the aforementioned concentrate (15), involving transesterification (17) of the fatty acids in the biomass with sulphuric acid as a catalyser and methanol as a reagent (16), to obtain the respective methyl esters making it up. When the reaction is complete, the biodiesel (20) is extracted from the reaction mixture using hexane (18), which is then evaporated to obtain the biodiesel (20).
- A stage consisting of biogas production, whereby the residual biomass (21) resulting from the previous stage is subjected to an anaerobic digestion process (22) by the action of methanogenic bacteria, in order to produce biogas (23) from the resulting methanol-free and hexane-free reaction mixture (21), whereby the residual carbonaceous matter contained in the same is transformed into methane, hydrogen and CO₂.

2. Photosynthetic microorganisms valorization process for integral use of biomass, according to claim 1, **characterized in that** at the bioethanol production stage (8), the saccharification (6) of the carbohydrates occurs under acidic conditions within a moderate pH of 1.5-2.5 and temperature range of 95-115°C.

3. Photosynthetic microorganisms valorization process for integral use of biomass, according to claim 1 or 2, **characterized in that** the yeast used for fermentation belongs to the genus *Saccharomyces* and that it is fermented for 24 h.

4. Photosynthetic microorganisms valorization process for integral use of biomass, according to claim 1, 2 or 3, **characterised in that** the alcoholic fermentation stage (7) forming part of the bioethanol production stage (8) is performed by cooling the concentrate to 30ºC and adjusting the pH to 8.

5. Photosynthetic microorganisms valorization process for integral use of biomass, according to any of claims 1-4, **characterized in that** the yeast is recovered for reuse by filtering and/or centrifuging, with bioethanol and unmetabolised sugar residues remaining as content in the liquid phase.

6. Photosynthetic microorganisms valorization process for integral use of biomass, according to claim 1, **characterized in that** the bioethanol (8) produced is separated by means of vacuum distillation and subsequent desiccation using molecular sieves.

7. Photosynthetic microorganisms valorization process for integral use of biomass, according to claim 1, **characterized in that** the enzymatic hydrolysis process (11) is performed using the residual biomass (9) in an stirred-tank reactor at a controlled pH of 7.7-8.1 and temperature of 40-55°C for approximately 4 h, under gentle conditions and without using toxic products or harsh conditions.

8. Photosynthetic microorganisms valorization process for integral use of biomass, according to claim 1, **characterized in that** the peptide and amino acid concentrate (10) is stabilised by acid or alkaline pH adjustment to prevent decomposition.

9. Photosynthetic microorganisms valorization process for integral use of biomass, according to claim 1, **characterized in that** the transesterification (17) is performed under gentle conditions without prior biomass drying, using sulphuric acid at 2% and at a temperature of 90-110ºC, for 2 h.

10. Photosynthetic microorganisms valorization process for integral use of biomass, according to claim 1, **characterized in that** at the biodiesel production stage (20), the unused methanol is removed from the reaction mixture by vacuum distillation.

11. Photosynthetic microorganisms valorization process for integral use of biomass, according to claim 1, **characterized in that** the anaerobic digestion process (22) forming part of the biogas production stage takes place in sealed tanks under anaerobic conditions at temperatures of 30-40ºC, with a residence time of approximately 5-8 days.

## Patentansprüche

1. Verfahren zur Verwertung photosynthetischer Mikroorganismen zur integrierten Verwendung von Biomasse, **dadurch gekennzeichnet, dass** es folgende Stufen einschließt:
- eine Anfangsstufe, bei der Mikroalgenbiomasse (1) in einen Rührtank eingebracht wird, in dem cellulolytische Enzyme (2) verwendet werden, um eine Verflüssigung der Zucker (3) herbeizuführen, wobei die Zellwände aufgebrochen und die löslichen Kohlenhydrate freigesetzt werden und die restlichen Bestandteile in der übrig gebliebenen Biomasse verbleiben; das erhaltene Gemisch unter Verwendung eines Filter- und/oder Zentrifugationsverfahrens (4) getrennt wird, wobei eine geklärte kohlenhydratreiche Lösung (5) und ein die übrig gebliebene Biomasse (9) enthaltendes Konzentrat erzeugt wird;
- eine Stufe, die in der Produktion von Bioethanol und anderen abgeleiteten Produkten, wie Milchsäure, aus der oben erwähnten Lösung (5) mittels Verzuckerung (6) der Kohlenhydrate gefolgt von einer alkoholischen Gärung (7) mit Hefe besteht, um die Monosaccharide in einem Gärungsprozess mit hoher Ausbeute in Bioethanol (8) zu verwandeln;
- eine Stufe, die in der Produktion von Peptid- und Aminosäurekonzentraten (10) aus der übrig gebliebenen Biomasse (9) unter Verwendung proteolytischer Enzyme (12), die die Proteine in einem enzymatischen Hydrolyseprozess (11) abbauen und auflösen, besteht, wobei die Proteine in Peptide und Aminosäuren mit niedrigerem Molekulargewicht umgewandelt werden, die für Lebensmittel oder hochwertige Düngemittel für die Landwirtschaft verwendet werden können; Das erhaltene Reaktionsgemisch wird anschließend unter Verwendung eines Filter- und/oder Zentrifugationsverfahrens (13) getrennt, wobei eine peptid- und aminosäurereiche Lösung (14) und ein die übrig gebliebene Biomasse (15) enthaltendes Konzentrat erzeugt wird.
- eine Stufe, die in der Biodieselproduktion aus dem oben erwähnten Konzentrat (15) besteht, wobei eine Umesterung (17) der Fettsäuren in der Biomasse mit Schwefelsäure als Katalysator und Methanol als Reagenz (16) stattfindet, um die entsprechenden Methylester, aus denen es besteht, zu erhalten; Nach Beendigung der Reaktion wird der Biodiesel (20) mithilfe von Hexan (18), das anschließend verdampft wird, aus dem Reaktionsgemisch extrahiert wird, um den Biodiesel (20) zu erhalten.
- eine Stufe, die in der Biogasproduktion besteht, wobei die übrig gebliebene aus der vorherigen Stufe stammende Biomasse (21) einem anaeroben Verdauungsprozess (22) unter Einwirkung von methanogenen Bakterien unterworfen wird, um aus dem resultierenden methanolfreien und hexanfreien Reaktionsgemisch (21) Biogas (23) herzustellen, wobei die übrig gebliebene darin enthaltene kohlenstoffhaltige Materie in Methan, Wasserstoff und CO₂ umgewandelt wird.

2. Verfahren zur Verwertung photosynthetischer Mikroorganismen zur integrierten Verwendung von Biomasse nach Anspruch 1, **dadurch gekennzeichnet, dass** die Verzuckerung (6) der Kohlenhydrate in der Stufe der Bioethanolproduktion (8) unter sauren Bedingungen bei einem moderaten pH-Wert von 1,5-2,5 und in einem Temperaturbereich von 95-115 °C erfolgt.

3. Verfahren zur Verwertung photosynthetischer Mikroorganismen zur integrierten Verwendung von Biomasse nach Anspruch 1 oder 2, **dadurch gekennzeichnet, dass** die zur Gärung eingesetzte Hefe zur Gattung *Saccaromyces* gehört und dass sie 24 Stunden lang gärt.

4. Verfahren zur Verwertung photosynthetischer Mikroorganismen zur integrierten Verwendung von Biomasse nach Anspruch 1, 2 oder 3, **dadurch gekennzeichnet, dass** die Stufe der alkoholischen Gärung (7), die Teil der Stufe der Bioethanolproduktion (8) ist, durch Abkühlen des Konzentrats auf 30 °C und Einstellen des pH-Werts auf 8 durchgeführt wird.

5. Verfahren zur Verwertung photosynthetischer Mikroorganismen zur integrierten Verwendung von Biomasse nach einem der Ansprüche 1-4, **dadurch gekennzeichnet, dass** die Hefe durch Filtern und/oder Zentrifugieren zur Wiederverwendung zurückgewonnen wird, wobei das Bioethanol und die nicht metabolisierten Zuckerreste als Gehalt in der Flüssigphase bleiben.

6. Verfahren zur Verwertung photosynthetischer Mikroorganismen zur integrierten Verwendung von Biomasse nach Anspruch 1, **dadurch gekennzeichnet, dass** das produzierte Bioethanol (8) durch Vakuumdestillation und anschließender Trocknung unter Verwendung von Molekularsieben abgetrennt wird.

7. Verfahren zur Verwertung photosynthetischer Mikroorganismen zur integrierten Verwendung von Biomasse nach Anspruch 1, **dadurch gekennzeichnet, dass** der enzymatische Hydrolyseprozess (11) in einem Rührtankreaktor unter Verwendung der übrig gebliebenen Biomasse (9) für etwa 4 h bei einem kontrollierten pH-Wert von 7,7-8,1 und einer Temperatur von 40-55 °C unter schonenden Bedingungen und ohne den Einsatz von toxischen Produkten oder rauen Bedingungen durchgeführt wird.

8. Verfahren zur Verwertung photosynthetischer Mikroorganismen zur integrierten Verwendung von Biomasse nach Anspruch 1, **dadurch gekennzeichnet, dass** das Peptid- und Aminosäurekonzentrat (10) durch Einstellen eines sauren oder alkalischen pH-Werts stabilisiert wird, um einer Zersetzung vorzubeugen.

9. Verfahren zur Verwertung photosynthetischer Mikroorganismen zur integrierten Verwendung von Biomasse nach Anspruch 1, **dadurch gekennzeichnet, dass** die Umesterung (17) für 2 h unter schonenden Bedingungen und ohne vorheriges Trocknen der Biomasse unter Verwendung von 2 %-iger Schwefelsäure bei einer Temperatur von 90-110 °C durchgeführt wird.

10. Verfahren zur Verwertung photosynthetischer Mikroorganismen zur integrierten Verwendung von Biomasse nach Anspruch 1, **dadurch gekennzeichnet, dass** in der Stufe der Biodieselproduktion (20) das nicht verwendete Methanol durch Vakuumdestillation aus dem Reaktionsgemisch entfernt wird.

11. Verfahren zur Verwertung photosynthetischer Mikroorganismen zur integrierten Verwendung von Biomasse nach Anspruch 1, **dadurch gekennzeichnet, dass** der anaerobe Verdauungsprozess (22), der Teil der Stufe der Biogasproduktion ist, unter anaeroben Bedingungen bei Temperaturen von 30-40 °C mit einer Verweilzeit von etwa 5-8 Tagen in versiegelten Tanks stattfindet.

## Revendications

1. Procédé de valorisation de micro-organismes photosynthétiques pour utilisation intégrale de biomasse, **caractérisé en ce qu'**il comprend les étapes suivantes :
- Une étape initiale par laquelle de la biomasse micro-algale (1) est placée dans une cuve avec agitateur dans laquelle des enzymes cellulolytiques (2) sont utilisées pour produire la liquéfaction des sucres (3), par rupture des parois cellulaires et libération des hydrates de carbone solubles, le reste des composants restant dans la biomasse résiduelle ; le mélange obtenu est séparé en utilisant un procédé de filtrage et/ou de centrifugation (4), générant une solution clarifiée (5) riche en hydrates de carbone et un concentré contenant la biomasse résiduelle (9).
- Une étape consistant en la production de bioéthanol et autres produits dérivés tels que l'acide lactique à partir de la solution mentionnée ci-dessus (5) par une saccharification (6) des hydrates de carbone, suivie d'une fermentation alcoolique (7) avec de la levure pour transformer les monosaccharides en bioéthanol (8) avec un procédé de fermentation à haut rendement.
- Une étape consistant à produire à partir de la biomasse résiduelle (9) des concentrés de peptides et d'acides aminés (10) en utilisant des enzymes protéolytiques (12) qui dégradent et solubilisent les protéines dans un procédé d'hydrolyse enzymatique (11), transformant les protéines en peptides et en acides aminés avec un poids moléculaire inférieur qui peuvent être utilisés pour des denrées alimentaires ou comme engrais agricoles de grande valeur. Le mélange de réaction obtenu est ensuite séparé en utilisant un procédé de filtrage et/ou de centrifugation (13), générant une solution (14) riche en peptides et en acides aminés et un concentré contenant la biomasse résiduelle (15).
- Une étape consistant en la production de biodiesel à partir du concentré mentionné ci-dessus (15), impliquant la transestérification (17) des acides gras dans la biomasse avec de l'acide sulfurique comme catalyseur et du méthanol comme réactif (16), pour obtenir les esters méthyliques respectifs qui le constituent. Lorsque la réaction est achevée, le biodiesel (20) est extrait du mélange de réaction en utilisant de l'hexane (18), qui est ensuite évaporé pour obtenir le biodiesel (20).
- Une étape consistant en la production de biogaz, par laquelle la biomasse résiduelle (21) résultant de l'étape précédente est soumise à un procédé de digestion anaérobie (22) par l'action de bactéries méthanogènes, afin de produire du biogaz (23) à partir du mélange de réaction sans méthanol et sans hexane obtenu (21), par laquelle la matière carbonée résiduelle contenue dans ce dernier est transformée en méthane, hydrogène et CO₂.

2. Procédé de valorisation de micro-organismes photosynthétiques pour utilisation intégrale de biomasse, selon la revendication 1, **caractérisé en ce que** lors de l'étape de production de bioéthanol (8), la saccharification (6) des hydrates de carbone se produit dans des conditions acides dans une fourchette de pH modéré de 1,5 à 2,5 et à une température allant de 95 à 115°C.

3. Procédé de valorisation de micro-organismes photosynthétiques pour utilisation intégrale de biomasse, selon la revendication 1 ou 2, **caractérisé en ce que** la levure utilisée pour la fermentation appartient au genre *Saccharomyces* et qu'elle fermente pendant 24 h.

4. Procédé de valorisation de micro-organismes photosynthétiques pour utilisation intégrale de biomasse, selon la revendication 1, 2 ou 3, **caractérisé en ce que** l'étape de fermentation alcoolique (7) faisant partie de l'étape de production de bioéthanol (8) est réalisée en refroidissant le concentré à 30°C et en ajustant le pH à 8.

5. Procédé de valorisation de micro-organismes photosynthétiques pour utilisation intégrale de biomasse, selon l'une quelconque des revendications 1-4, **caractérisé en ce que** la levure est récupérée pour réutilisation en filtrant et/ou en centrifugeant, avec du bioéthanol et des résidus de sucre non métabolisé restant comme contenu dans la phase liquide.

6. Procédé de valorisation de micro-organismes photosynthétiques pour utilisation intégrale de biomasse, selon la revendication 1, **caractérisé en ce que** le bioéthanol (8) produit est séparé au moyen d'une distillation sous vide suivie d'une dessiccation en utilisant des tamis moléculaires.

7. Procédé de valorisation de micro-organismes photosynthétiques pour utilisation intégrale de biomasse, selon la revendication 1, **caractérisé en ce que** le procédé d'hydrolyse enzymatique (11) est réalisé en utilisant la biomasse résiduelle (9) dans un réacteur agité à un pH contrôlé de 7,7-8,1 et une température de 40-55°C pendant environ 4 h, dans des conditions douces et sans utiliser de produits toxiques ni de conditions dures.

8. Procédé de valorisation de micro-organismes photosynthétiques pour utilisation intégrale de biomasse, selon la revendication 1, **caractérisé en ce que** le concentré de peptides et d'acides aminés (10) est stabilisé par un ajustement du pH acide ou alcalin pour empêcher la décomposition.

9. Procédé de valorisation de micro-organismes photosynthétiques pour utilisation intégrale de biomasse, selon la revendication 1, **caractérisé en ce que** la transestérification (17) est réalisée dans des conditions douces sans séchage préalable de la biomasse, en utilisant de l'acide sulfurique à 2% et à une température de 90-110°C, pendant 2 h.

10. Procédé de valorisation de micro-organismes photosynthétiques pour utilisation intégrale de biomasse, selon la revendication 1, **caractérisé en ce que** lors de l'étape de production de biodiesel (20), le méthanol inutilisé est retiré du mélange de réaction par distillation sous vide.

11. Procédé de valorisation de micro-organismes photosynthétiques pour utilisation intégrale de biomasse, selon la revendication 1, **caractérisé en ce que** le procédé de digestion anaérobie (22) faisant partie de l'étape de production de biogaz a lieu dans des cuve fermées hermétiquement dans des conditions anaérobies à des températures de 30-40°C, avec un temps de séjour d'environ 5-8 jours.
